(19) Europäisches Patentamt
European Patent Office
Office européen des brevets

(11) **EP 3 050 484 A1**

(12) **EUROPEAN PATENT APPLICATION**
published in accordance with Art. 153(4) EPC

(43) Date of publication:
**03.08.2016 Bulletin 2016/31**

(51) Int Cl.:
**A61B 1/00** (2006.01)

(21) Application number: **13894698.3**

(22) Date of filing: **24.09.2013**

(86) International application number:
**PCT/JP2013/075625**

(87) International publication number:
**WO 2015/044996 (02.04.2015 Gazette 2015/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Olympus Corporation**
**Hachioji-shi**
**Tokyo 192-8507 (JP)**

(72) Inventor: **MORITA, Yasunori**
**Hachioji-shi**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **ENDOSCOPE DEVICE AND METHOD FOR CONTROLLING ENDOSCOPE DEVICE**

(57)    An endoscope apparatus includes: an image acquisition section that acquires a plurality of *in vivo* images that were obtained by capturing an *in vivo* object using an imaging optical system; an in-focus evaluation value calculation section (contrast value calculation section 336) that calculates an in-focus evaluation value corresponding to each of the plurality of *in vivo* images; a focus control section (lens position control section 320) that controls a focus operation of the imaging optical system based on the in-focus evaluation value; and a freeze image setting section (337) that selects at least one *in vivo* image from the plurality of *in vivo* images based on the degree of in-focus represented by the in-focus evaluation value, and sets the selected at least one *in vivo* image to be a freeze image.

FIG. 7

## Description

TECHNICAL FIELD

The present invention relates to an endoscope apparatus, a method for controlling an endoscope apparatus, and the like.

BACKGROUND ART

An imaging device such as an endoscope is desired to generate a deep-focus image so that the doctor can easily make a diagnosis. Therefore, the depth of field of an endoscope is increased by utilizing an optical system that has a relatively large F-number. In recent years, an image sensor having about several hundred thousand pixels has been used for an endoscope system. Since an image sensor having a large number of pixels has a small pixel pitch and a small permissible circle of confusion, the depth of field of the imaging device decreases since it is necessary to decrease the F-number. In this case, it is difficult to provide a deep-focus image.

For example, Patent Document 1 discloses an endoscope apparatus that is configured so that a driver section that drives the lens position of an objective optical system is provided to an imaging section of the endoscope to implement an autofocus (hereinafter may be referred to as "AF") process on the object.

RELATED-ART DOCUMENT

PATENT DOCUMENT

Patent Document 1: JP-A-8-106060

SUMMARY OF THE INVENTION

TECHNICAL PROBLEM

When a doctor who performs an endoscopic examination desires to closely observe the attention area (i.e., area of interest), the doctor acquires a freeze image (still image) by operating a freeze switch or the like, and closely observes the attention area using the freeze image. The freeze image may be acquired using an endoscope that performs an AF process. In this case, since the AF process is performed by moving the objective lens of the optical system in the direction in which the object contour signal increases to even only a small extent, an image in which the object is out of focus is generated until the AF process is completed. Therefore, when the object is out of focus within the acquired freeze image, the doctor must operate the freeze switch again. As a result, the doctor must perform a complex operation.

Several aspects of the invention may provide an endoscope apparatus, a method for controlling an endoscope apparatus, and the like that can display a freeze image in which the object is in focus.

SOLUTION TO PROBLEM

According to one aspect of the invention, an endoscope apparatus includes:

an image acquisition section that acquires a plurality of *in vivo* images that were obtained by capturing an *in vivo* object using an imaging optical system, each of the plurality of *in vivo* images including an image of the *in vivo* object;
an in-focus evaluation value calculation section that calculates an in-focus evaluation value that represents a degree of in-focus corresponding to each of the plurality of *in vivo* images;
a focus control section that controls a focus operation of the imaging optical system based on the in-focus evaluation value; and
a freeze image setting section that selects at least one *in vivo* image from the plurality of *in vivo* images based on the degree of in-focus represented by the in-focus evaluation value, and sets the selected at least one *in vivo* image to be a freeze image.

According to one aspect of the invention, the focus operation is controlled based on the in-focus evaluation value, and at least one *in vivo* image among the plurality of *in vivo* images is set to be the freeze image based on the degree of in-focus represented by the in-focus evaluation value. This makes it possible to display a freeze image in which the object is in focus.

According to one aspect of the invention, a method for controlling an endoscope apparatus includes:

acquiring a plurality of *in vivo* images that were obtained by capturing an *in vivo* object using an imaging optical system, each of the plurality of *in vivo* images including an image of the *in vivo* object;
calculating an in-focus evaluation value that represents a degree of in-focus corresponding to each of the plurality of *in vivo* images;
controlling a focus operation of the imaging optical system based on the in-focus evaluation value; and
selecting at least one *in vivo* image from the plurality of *in vivo* images based on the degree of in-focus represented by the in-focus evaluation value, and setting the selected at least one *in vivo* image to be a freeze image.

BRIEF DESCRIPTION OF DRAWINGS

FIG. 1 illustrates a configuration example of an endoscope apparatus according to a first embodiment.
FIG. 2 illustrates a detailed configuration example of a rotary color filter.
FIG. 3 illustrates an example of the spectral characteristics of a color filter.

FIG. 4 is a view illustrating the relationship between the position of a focus lens and an in-focus object plane position.

FIG. 5 is a view illustrating a depth of field when an in-focus object plane position is situated on a near point side.

FIG. 6 is a view illustrating a depth of field when an in-focus object plane position is situated on a far point side.

FIG. 7 illustrates a detailed configuration example of an image processing section.

FIG. 8 is a view illustrating an area division process performed by an attention area setting section.

FIG. 9 is a view illustrating an operation performed by a freeze image setting section.

FIG. 10 is a view illustrating a modification of an operation performed by a freeze image setting section.

FIG. 11 is a view illustrating a second modification of an operation performed by a freeze image setting section.

FIG. 12 illustrates an example of a flowchart of an operation performed by a lens position control section.

FIGS. 13A and 13B illustrate an example of a display image when two freeze candidate images are displayed.

FIG. 14 illustrates a configuration example of an endoscope apparatus according to a second embodiment.

FIG. 15 is a view illustrating an operation performed by a freeze image setting section.

DESCRIPTION OF EMBODIMENTS

Exemplary embodiments of the invention are described below. Note that the following exemplary embodiments do not in any way limit the scope of the invention laid out in the claims. Note also that all of the elements described below in connection with the exemplary embodiments should not necessarily be taken as essential elements of the invention.

1. Outline

An outline of several embodiments of the invention is described below. The depth of field of an endoscope apparatus becomes shallow as the number of pixels of an image sensor increases, and it becomes difficult to bring the desired object into focus. In particular, the depth of field of an endoscope apparatus that implements zoom observation further becomes shallow as the imaging magnification of an imaging section increases, or the distance from the imaging section to the object decreases. In such a case, the object easily lies outside the depth-of-field range even when the position of the object has changed (i.e., the object has moved) to only a small extent.

When a doctor desires to closely observe an attention area, the doctor displays a freeze image (still image) on a display section by operating a freeze switch provided to an operation section. In this case, the object included in the attention area easily lies outside the depth-of-field range when the depth of field is shallow. Therefore, it may be necessary for the doctor to repeatedly operate the freeze switch in order to obtain an image in which the object included in the attention area is in focus (i.e., it is troublesome).

For example, a continuous AF process may be used to prevent a situation in which the object becomes out of focus. However, since the continuous AF process performs a wobbling operation, the focus lens moves little by little in the forward-backward direction in a state in which the object is in focus. Therefore, a freeze image in which the object is in focus is not necessarily obtained the timing at which the freeze switch was pressed.

According to several embodiments of the invention, captured images corresponding to a plurality of frames are stored, and a captured image among the stored captured images in which the object is in focus is displayed on a display section as a freeze image. According to this configuration, the user can easily obtain a freeze image in which the object is in focus by merely pressing the freeze switch without taking account of whether or not the object is in focus (i.e., the operation performed by the user is facilitated).

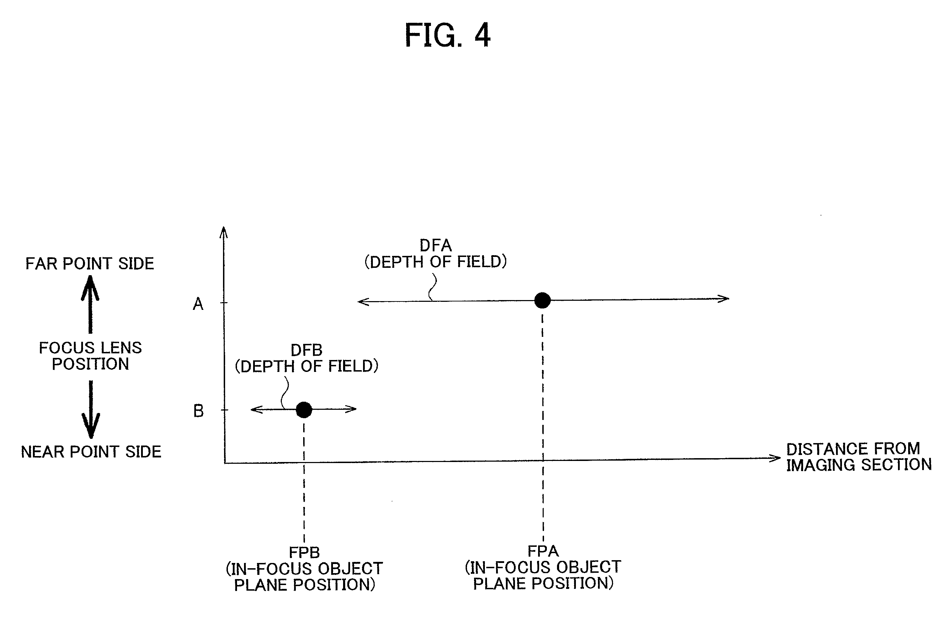

A first embodiment illustrates a basic configuration and method. The first embodiment illustrates an example in which a dual focus switch process is used as described later with reference to FIG. 4. The dual focus switch process has an advantage in that the AF mechanism can be normally simplified. On the other hand, the degree of freedom relating to the focus adjustment process may decrease, and it may be difficult to implement a fine focus operation (i.e., it may be difficult to obtain a freeze image in which the object is in focus). According to the first embodiment, however, a captured image in which the object is in focus is selected as the freeze image from the captured images corresponding to a plurality of frames, it is possible to obtain a freeze image in which the object is in focus even when the dual focus switch process is used. A second embodiment illustrates an example in which a continuous AF process is used. Since the continuous AF process has a higher degree of freedom relating to the focus adjustment process as compared with the dual focus switch process, it is possible to implement a fine focus operation.

2. First embodiment (dual focus switch)

2.1. Endoscope apparatus

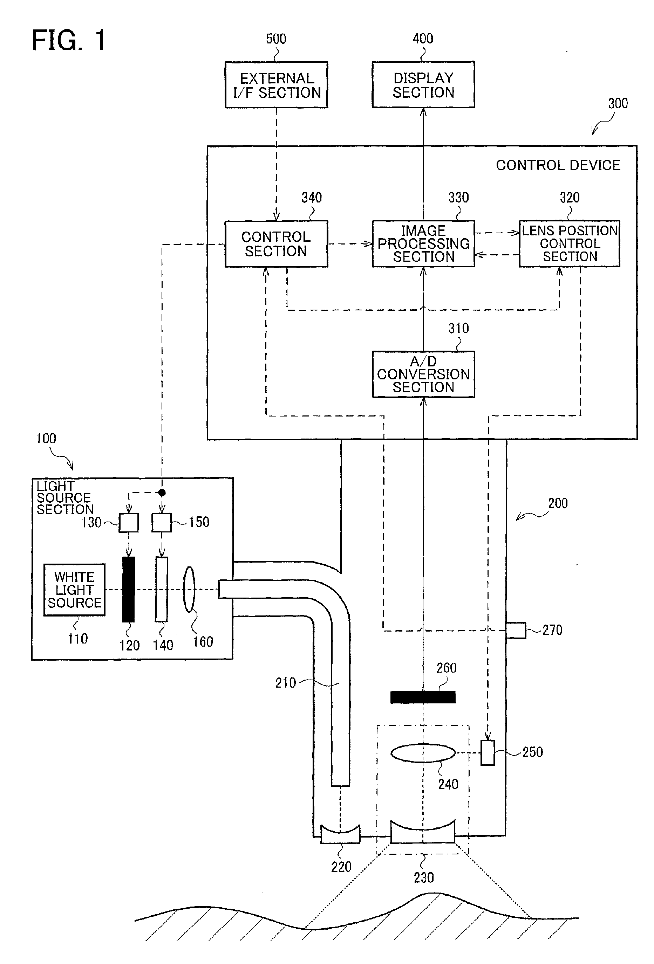

FIG. 1 illustrates a configuration example of an endoscope apparatus according to the first embodiment. The endoscope apparatus includes a light source section 100, an imaging section 200, a control device 300 (processor section (processor)), a display section 400, and an external I/F section 500.

The light source section 100 includes a white light source 110, a light source aperture 120, a light source aperture driver section 130 that drives the light source aperture 120, and a rotary color filter 140 that includes a plurality of filters that differ in spectral transmittance. The light source section 100 also includes a rotation driver section 150 that drives the rotary color filter 140, and a condenser lens 160 that focuses the light that has passed through the rotary color filter 140 on the incident end face of a light guide fiber 210.

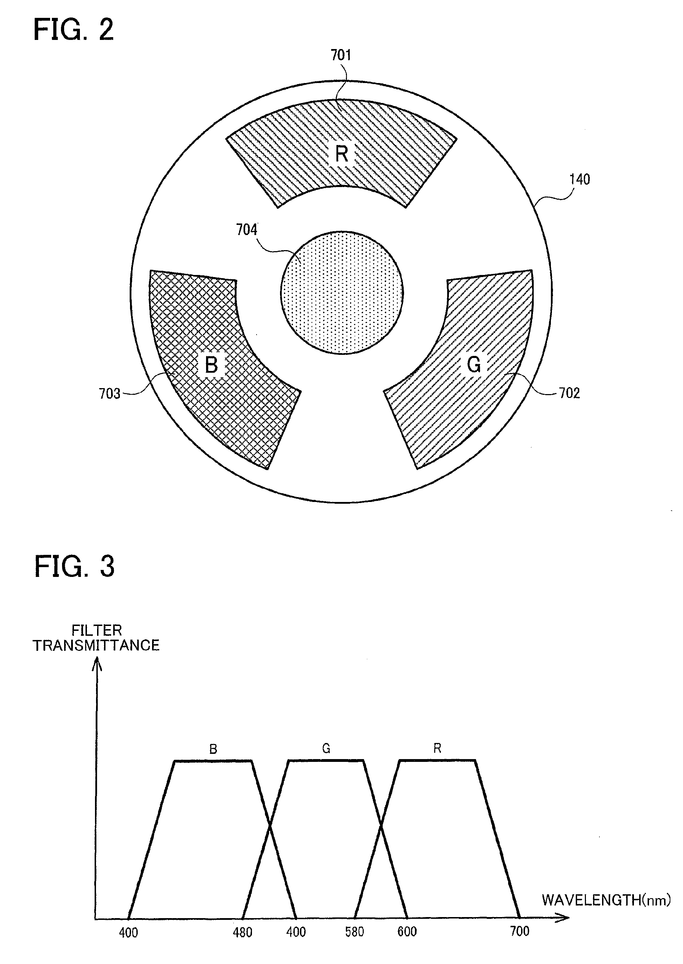

The light source aperture driver section 130 adjusts the intensity of illumination light by opening and closing the light source aperture 120 based on a control signal output from a control section 340 included in the control device 300. FIG. 2 illustrates a detailed configuration example of the rotary color filter 140. The rotary color filter 140 includes a red (R) color filter 701, a green (G) color filter 702, a blue (B) color filter 703, and a rotary motor 704. FIG. 3 illustrates an example of the spectral characteristics of the color filters 701 to 703. The rotation driver section 150 rotates the rotary color filter 140 at a given rotational speed in synchronization with the imaging period of an image sensor 260 based on the control signal output from the control section 340. For example, when the rotary color filter 140 is rotated at 20 revolutions per second, each color filter crosses the incident white light every 1/60th of a second. In this case, the image sensor 260 captures and transfers an image signal every 1/60th of a second. The image sensor 260 is a monochrome single-chip image sensor, for example. The image sensor 260 is implemented by a CCD image sensor or a CMOS image sensor, for example. Specifically, the endoscope apparatus according to the first embodiment frame-sequentially captures an R image, a G image, and a B image every 1/60th of a second.

The imaging section 200 is formed to be elongated and flexible so that the imaging section 200 can be inserted into a body cavity, for example. The imaging section 200 includes the light guide fiber 210 that guides the light focused by the light source section 100 to an illumination lens 220, and the illumination lens 220 that diffuses the light guided by the light guide fiber 210 to illuminate the observation target. The imaging section 200 also includes an objective lens 230 that focuses the reflected light from the observation target, a focus lens 240 (focus adjustment lens) that adjusts the focal distance, a switch section 250 that switches the position of the focus lens 240 between discrete positions, and the image sensor 260 that detects the focused reflected light.

The switch section 250 is a voice coil motor (VCM), for example. The switch section 250 is connected to the focus lens 240. The switch section 250 switches the position of the focus lens 240 between a plurality of discrete positions to discretely adjust the in-focus object plane position (i.e., the position of the object at which the object is in focus). The relationship between the position of the focus lens 240 and the in-focus object plane position is described later with reference to FIG. 4.

The imaging section 200 is provided with a freeze switch 270 that allows the user to issue a freeze instruction. The user can input and cancel a freeze instruction signal by operating the freeze switch 270. When the user has issued the freeze instruction by operating the freeze switch 270, the freeze instruction signal is output from the freeze switch 270 to the control section 340.

The control device 300 controls each section of the endoscope apparatus, and performs image processing. The control device 300 includes an A/D conversion section 310, a lens position control section 320 (focus control section in a broad sense), an image processing section 330, and the control section 340.

The image signal that has been converted into a digital signal by the A/D conversion section 310 is transmitted to the image processing section 330. The image signal is processed by the image processing section 330, and transmitted to the display section 400. The image processing section 330 transmits a contrast value calculated from the image signal to the lens position control section 320. The lens position control section 320 transmits a control signal to the switch section 250 to change the position of the focus lens 240. The lens position control section 320 transmits a control signal that represents the position of the focus lens 240 to the image processing section 330. The control section 340 controls each section of the endoscope apparatus. More specifically, the control section 340 synchronizes the light source aperture driver section 130, the lens position control section 320, and the image processing section 330. The control section 340 is connected to the freeze switch 270 and the external I/F section 500, and transmits the freeze instruction signal to the image processing section 330. The control section 340 transmits an aperture value L that represents the degree of opening of the light source aperture to the lens position control section 320.

The display section 400 is a display device that can display a video (moving image), and is implemented by a CRT, a liquid crystal monitor, or the like.

The external I/F section 500 is an interface that allows the user to input information to the endoscope apparatus, for example. The external I/F section 500 may include a freeze button (not illustrated in the drawings) that allows the user to issue the freeze instruction. In this case, the user can issue the freeze instruction using the external I/F section 500. Note that the function of the freeze button is the same as that of the freeze switch 270 provided to the imaging section 200. The external I/F section 500 outputs the freeze instruction signal to the control section 340. The external I/F section 500 also includes a power switch (power ON/OFF switch), a mode (e.g., imaging mode) switch button, and the like.

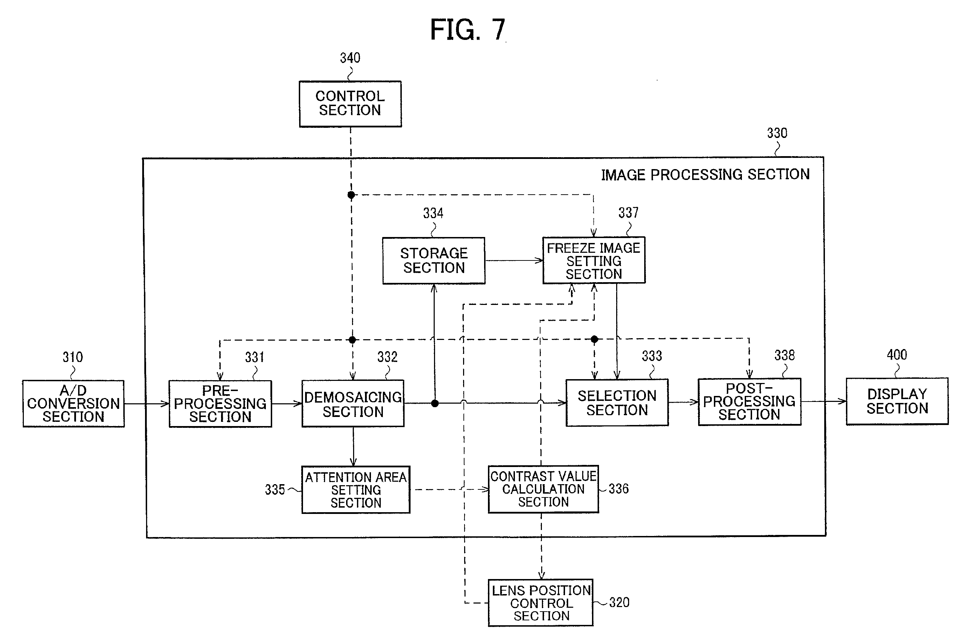

The image processing section 330 performs image processing on the captured image that has been converted into a digital signal by the A/D conversion section 310. More specifically, the image processing section 330 performs pre-processing, a demosaicing process, a contrast value (in-focus evaluation value in a broad sense) calculation process, a freeze image selection process, post-processing, and the like. The image processing section 330 outputs the freeze image or the video (captured image) subjected to post-processing to the display section 400, and outputs the contrast value to the lens position control section 320. The details of the image processing section 330 are described later with reference to FIG. 7.

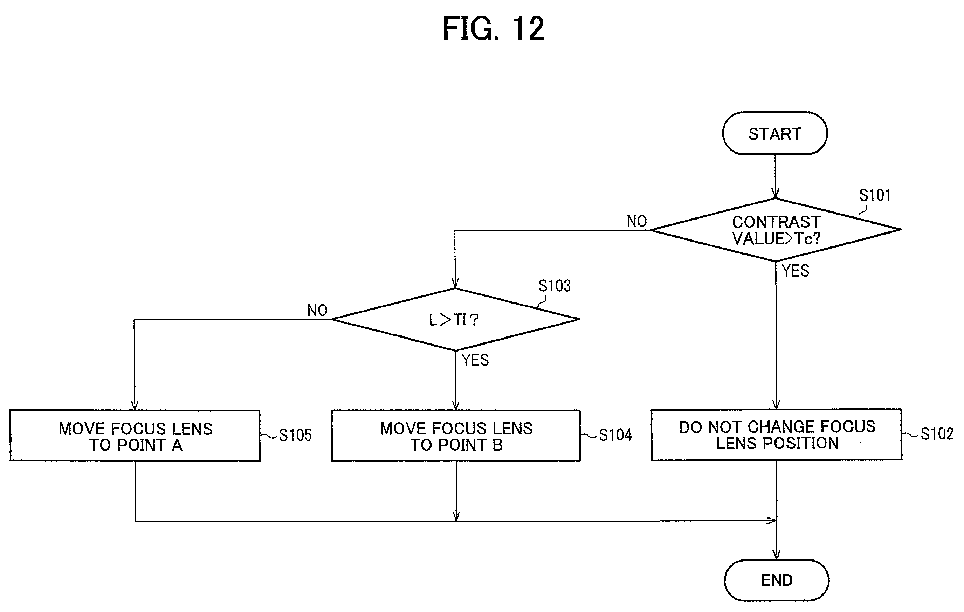

The lens position control section 320 controls the switch section 250 based on the contrast value input from the image processing section 330, and the aperture value L of the light source aperture input from the control section 340. The switch section 250 switches the position of the focus lens 240 based on the instruction from the lens position control section 320 to implement an AF control process. The details of the lens position control section 320 are described later with reference to FIG. 12.

Although an example in which a frame-sequential imaging method is used has been described above, the configuration is not limited thereto. For example, an imaging method that utilizes a primary-color Bayer image sensor, a single-ship complementary-color image sensor, a double-chip primary-color image sensor, a triple-chip primary-color image sensor, or the like may also be used. Although an example of normal light observation that utilizes white light as the illumination light has been described above, the configuration is not limited thereto. For example, it is also possible to implement special light observation such as narrow band imaging (NBI) that utilizes light having a band narrower than that of white light as the illumination light.

2.2. Relationship between position of focus lens and in-focus object plane position

The relationship between the position of the focus lens 240 and the in-focus object plane position is described below with reference to FIG. 4. In the first embodiment, the position of the focus lens 240 is switched between discrete lens positions A and B, and the in-focus object plane position is switched between positions FPA and FPB (see FIG. 4).

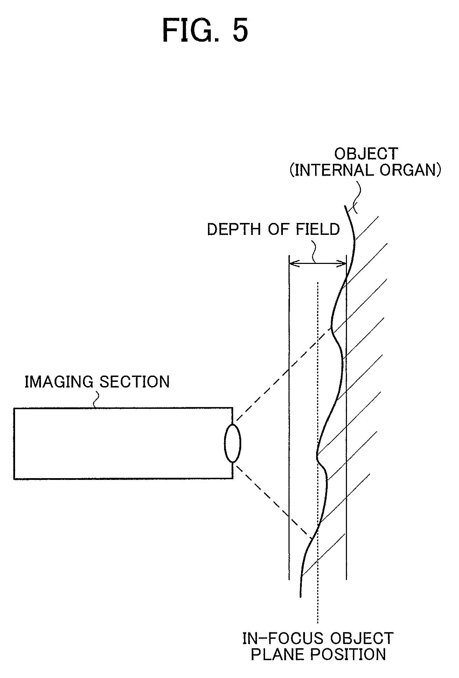

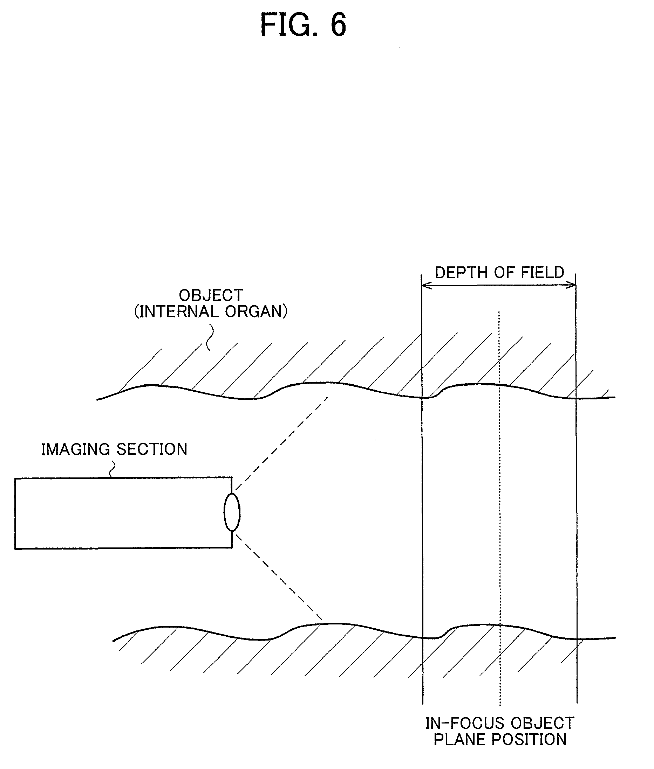

More specifically, the position of the focus lens 240 is switched between the point A that corresponds to the point FPA (hereinafter referred to as "far point") at which the in-focus object plane position is situated away from the imaging section 200, and the point B that corresponds to the point FPB (hereinafter referred to as "near point") at which the in-focus object plane position is situated close to the imaging section 200. The depth of field is normally shallow when the near point-side in-focus object plane position is selected, and the object easily lies outside the depth of field even when the object has moved to only a small extent. Therefore, the near point-side in-focus object plane position is suitable for closely observing a shallow object (see FIG. 5). On the other hand, the depth of field is deep when the far point-side in-focus object plane position is selected. Therefore, the far point-side in-focus object plane position is suitable for screening a hollow tubular object (see FIG. 6). The depth of field required for endoscopic observation is achieved by changing the position of the focus lens. For example, the range of the depth of field that can be achieved by combining a depth of field DFA (when the position of the focus lens 240 is set to the point A) and a depth of field DFB (when the position of the focus lens 240 is set to the point B) includes 2 to 70 mm.

The term "in-focus object plane position" used herein refers to the position of the object at which the object is in focus from the imaging section 200. For example, the in-focus object plane position is represented by the distance from the end of the imaging section 200 to the object along the optical axis of the imaging optical system. More specifically, the term "in-focus object plane position" used herein refers to the position of the object plane that corresponds to the image plane when the light-receiving plane of the image sensor 260 coincides with the image plane. Since it is considered that the object is in focus as long as the object lies within the depth of field of the imaging section 200, the in-focus object plane position may be set to an arbitrary position within the depth of field. For example, the in-focus object plane position FPA and the in-focus object plane position FPB illustrated in FIG. 4 may be set to an arbitrary position within the depth of field DFA and an arbitrary position within the depth of field DFB, respectively. In this case, the in-focus object plane position and the depth of field are also changed by changing the position of the focus lens 240.

The term "position" used herein in connection with the focus lens 240 refers to the position of the focus lens 240 within the imaging optical system. For example, the position of the focus lens 240 is represented by the distance from a reference point within the imaging optical system to the focus lens 240. The reference point may be the position of the lens of the imaging optical system that is situated closest to the object, the position of the light-receiving plane of the image sensor 260, or the like.

Although FIG. 4 illustrates an example in which the AF control process switches the in-focus object plane position between two in-focus object plane positions (dual focus switch process), the configuration is not limited thereto. For example, the AF control process may switch the in-focus object plane position between three or more discrete in-focus object plane positions.

Although an example in which the focus lens 240 is used as a lens that adjusts the focus (focus adjustment lens) has been described above, the configuration is not limited thereto. Specifically, the focus adjustment lens may be a focus lens when a zoom lens and a focus lens are driven independently, or may be a zoom lens when a zoom lens has a zoom magnification adjustment function and a focus adjustment function.

2.3. Image processing section

FIG. 7 illustrates a detailed configuration example of the image processing section 330 according to the first embodiment. The image processing section 330 includes a pre-processing section 331, a demosaicing section 332, a selection section 333, a storage section 334, an attention area setting section 335, a contrast value calculation section 336, a freeze image setting section 337, and a post-processing section 338.

The A/D conversion section 310 is connected to the pre-processing section 331. The pre-processing section 331 is connected to the demosaicing section 332. The demosaicing section 332 is connected to the selection section 333, the storage section 334, and the attention area setting section 335. The selection section 333 is connected to the post-processing section 338. The storage section 334 is connected to the freeze image setting section 337. The attention area setting section 335 is connected to the contrast value calculation section 336. The contrast value calculation section 336 is connected to the freeze image setting section 337 and the lens position control section 320. The lens position control section 320 is connected to the freeze image setting section 337. The freeze image setting section 337 is connected to the selection section 333. The post-processing section 338 is connected to the display section 400. The control section 340 is bidirectionally connected to each section, and controls each section.

The pre-processing section 331 performs an OB clamp process, a gain control process, and a WB correction process on the image signal input from the A/D conversion section using an OB clamp value, a gain control value, and a WB coefficient stored in the control section 340 in advance. The pre-processing section 331 transmits the resulting image signal to the demosaicing section 332.

The demosaicing section 332 performs a demosaicing process on the frame-sequential image signal processed by the pre-processing section 331 based on the control signal output from the control section 340. More specifically, the demosaicing section 332 stores the image signals that have been input frame-sequentially and correspond to each color (light) (R, G, or B) on a frame basis, and simultaneously reads the stored image signals that correspond to each color (light). Specifically, the demosaicing section 332 performs the demosaicing process on the R image, the G image, and the B image to obtain the captured image that corresponds to one frame. For example, when the R image, the G image, the B image, the R image, and the G image are input sequentially, the demosaicing section 332 sequentially performs the demosaicing process on the R image, the G image, and the B image, performs the demosaicing process on the G image, the B image, and the R image, and performs the demosaicing process on the B image, the R image, and the G image, and sequentially outputs the captured images that correspond to three frames.

When the rotary color filter 140 (see FIG. 1) is rotated at 20 revolutions per second, the captured images that correspond to 60 frames are obtained by the demosaicing process within 1 second. The demosaicing section 332 transmits the captured image (image signal) obtained by the demosaicing process to the selection section 333 and the attention area setting section 335. The demosaicing section 332 transmits the captured image to the storage section 334 based on the freeze instruction signal input from the control section 340. More specifically, when the freeze instruction signal has been input from the control section 340, the demosaicing section 332 stops transmitting the captured image to the storage section 334. When the freeze instruction signal has been canceled, the demosaicing section 332 transmits the captured image to the storage section 334.

The storage section 334 includes a frame memory that can store the captured images (transmitted from the demosaicing section 332) that correspond to a plurality of frames. For example, the storage section 334 includes a frame memory that can store the captured images that correspond to N frames (N is a natural number equal to or larger than 2) in time series. The storage section 334 sequentially stores the input captured images. When the captured image that corresponds to the (N+1)th frame has been input, the storage section 334 deletes the oldest captured image stored therein, and stores the input captured image.

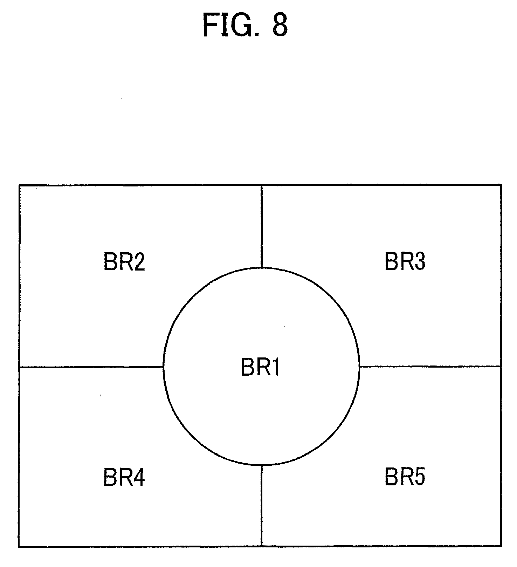

The attention area setting section 335 sets the attention area used to calculate the contrast value to the captured image transmitted from the demosaicing section 332. As illustrated in FIG. 8, the attention area setting section 335 divides the captured image into first to fifth areas BR1 to BR5 (a plurality of areas in a broad sense), and calculates brightness information about each area, for example. The brightness information represents a value obtained by adding up the brightness value of each pixel included in each area, for example. The attention area setting section 335 determines whether or not the brightness information about each area is equal to or larger than a threshold value, and sets an area for which the brightness information is equal to or larger than the threshold value to be the attention area. The attention area setting section 335 transmits information about the attention area and the captured image to the contrast value calculation section 336. When it is impossible to set the attention area (i.e., the brightness information about each area is less than the threshold value) (e.g., when the captured image has insufficient brightness), the attention area setting section 335 transmits a control signal that represents that the attention area is not present to the contrast value calculation section 336.

Although an example in which an area for which the brightness information (brightness) is equal to or larger than the threshold value is set to be the attention area has been described above, the configuration is not limited thereto. For example, the brightest area among the areas BR1 to BR5 may be set to be the attention area. Alternatively, the user may set the attention area in advance through the external I/F section 500. When the attention area is not set, the entire screen may be set to be the attention area. The attention area setting section 335 may include an attention area detection section that detects an area (e.g., lesion area) that has a specific feature quantity as compared with the peripheral area, and track the attention area detected by the attention area detection section. Alternatively, the center of the screen, the side opposite to a dark area (e.g., the area BR5 is set to be the attention area when the area BR2 is the darkest area (see FIG. 8), and the brightest area among the areas (peripheral areas) BR2 to BR5 is set to be the attention area when the center area (area BR1) is the darkest area), a lesion area (e.g., reddening, discoloration, or special light), an area that has a feature quantity (e.g., red) that differs from that of the peripheral area, an area that changes in brightness to only a small extent with the passing of time, or the like may be set to be the attention area, for example.

The contrast value calculation section 336 calculates the contrast value of the attention area from the information about the attention area and the captured image. For example, the contrast value calculation section 336 may calculate the contrast value that corresponds to an arbitrary channel from the captured image transmitted from the attention area setting section 335. Alternatively, the contrast value calculation section 336 may generate a brightness signal from the R-channel pixel value, the G-channel pixel value, and the B-channel pixel value, and calculate the contrast value from the pixel value of the brightness signal. For example, the contrast value calculation section 336 performs an arbitrary high-pass filtering process on each pixel included in the attention area, and calculates the contrast value by adding up the high-pass filter output value of each pixel within the attention area. When the control signal that represents that the attention area is not set to the captured image has been input, the contrast value calculation section 336 sets the contrast value to 0. The contrast value calculation section 336 transmits the contrast value of the attention area to the freeze image setting section 337 and the lens position control section 320.

Note that the contrast value calculation section 336 may include a bright spot-removing section. For example, the bright spot-removing section performs a threshold process on an arbitrary channel of each pixel included in the attention area or the pixel value of the brightness signal, and determines that a pixel for which the pixel value is equal to or larger than a threshold value is a bright spot. The contrast value calculation section 336 excludes the pixel that has been determined to be a bright spot from the target of the addition process. In this case, it is possible to reduce the effect of a bright spot on the contrast value.

Although an example in which a value obtained by adding up the high-pass filter output values is used as the contrast value has been described above, the configuration is not limited thereto. For example, the number of pixels for which the high-pass filter output value is equal to or larger than a threshold value may be calculated as the contrast value. In this case, the contrast value can be used as a value that represents the extent of the area in which the object is in focus.

The freeze image setting section 337 extracts the captured image in which the object is in focus from a plurality of captured images stored in the storage section 334 when the freeze instruction signal has been input from the control section 340. More specifically, the freeze image setting section 337 includes a memory (not illustrated in the drawings) that can store N contrast values input from the contrast value calculation section 336 and N positions of the focus lens 240 input from the lens position control section 320 in time series in a linked state. The memory included in the freeze image setting section 337 stores N contrast values and N lens positions that have been input at a timing that precedes the freeze instruction signal input timing when the freeze instruction signal has been input from the control section 340.

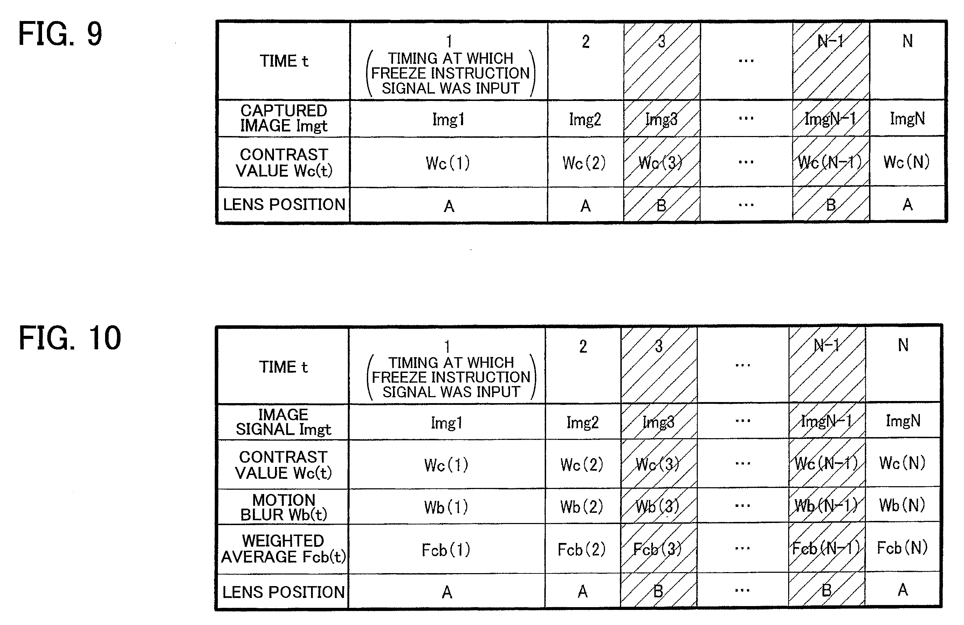

As illustrated in FIG. 9, the time t at which the freeze instruction signal was input is referred to as t=1, and the time t at which the captured image that corresponds to the frame that precedes the time t by N frames was input is referred to as t=N. A captured image Imgt stored in the storage section 334 at the time t, a contrast value Wc(t) of the attention area within the captured image Imgt, and a position A or B of the focus lens 240 when the captured image Imgt was captured are stored in the memory included in the freeze image setting section 337 in a linked state.

The freeze image setting section 337 detects the position of the focus lens 240 when the freeze instruction signal was input (time t=1) as a reference lens position (position A in FIG. 9), and determines the captured images that were captured at the same lens position as the reference lens position to be a freeze candidate image (i.e., the captured images that are not hatched in FIG. 9). The freeze image setting section 337 extracts the captured image having the largest contrast value from the freeze candidate images as a freeze image, and transmits the freeze image to the selection section 333.

Although an example in which the lens position at the time t=1 is used as the reference lens position has been described above, the configuration is not limited thereto. The lens position at the time t=M (M is a natural number that satisfies 1<M<N) may be used as the reference lens position. The time t=M is a value that is determined taking account of a time lag when the user performs the freeze operation. For example, the time t=M is a value that is proportional to the frame rate of the captured image.

Although an example in which the captured image having the largest contrast value is extracted as the freeze image has been described above, the configuration is not limited thereto. For example, the freeze image setting section 337 may detect a motion blur Wb(t) that represents the amount of blur of the captured image from the correlation between the captured image Imgt and the captured image Imgt+1, and calculate a weighted average Fcb(t) of the contrast value Wc(t) and the motion blur Wb(t) using the following expression (1) (see FIG. 10). The freeze image setting section 337 may set the captured image having the largest weighted average Fcb(t) among the captured images that were captured at the same lens position as the reference lens position (position A in FIG. 10) to be the freeze image. In this case, it is possible to set the captured image that has a large contrast value and a small amount of blur to be the freeze image.

$$Fcb(t)=a \times Wc(t)+b \times Wb(t) \qquad (1)$$

Note that a is a constant that satisfies $a \geq 0$, and b is a constant that satisfies $b \leq 0$. For example, a value input in advance from the outside, a value set in advance to the control section 340, or the like is used as the constants a and b.

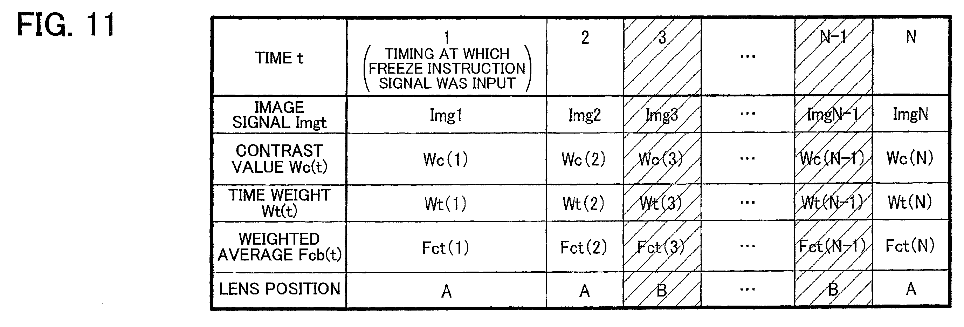

Alternatively, the freeze image setting section 337 may set a time weight Wt(t) that increases toward the time t=1, and calculate a weighted average Fct(t) of the contrast value Wc(t) and the time weight Wt(t) using the following expression (2) (see FIG. 11). The freeze image setting section 337 may set the captured image having the largest weighted average Fct(t) among the captured images that were captured at the same lens position as the reference lens position (position A in FIG. 11) to be the freeze image. In this case, it is possible to set the captured image that has a large contrast value and was captured at a timing closer to the timing at which the user performed the freeze operation to be the freeze image.

$$Fct(t)=c \times Wc(t)+d \times Wt(t) \qquad (2)$$

Note that c is a constant that satisfies $c \geq 0$, and d is a constant that satisfies $d \geq 0$. A value input in advance from the outside, a value set in advance to the control section 340, or the like is used as the constants c and d.

The selection section 333 selects the image that is transmitted to the post-processing section 338 based on the control signal from the control section 340. More specifically, when the freeze instruction signal has been input from the control section 340, the selection section 333 transmits the freeze image input from the freeze image setting section 337 to the post-processing section 338. When the freeze instruction signal has been canceled by the control section 340, the selection section 333 transmits the captured image input from the demosaicing section 332 to the post-processing section 338.

The post-processing section 338 performs a grayscale transformation process, a color process, and a contour enhancement process on the image transmitted from the selection section 333 using a grayscale transformation coefficient, a color conversion coefficient, and a contour enhancement coefficient stored in the control section 340 in advance. The post-processing section 338 transmits the resulting image to the display section 400.

2.4. Lens position control section

An example of the process performed by the lens position control section 320 is described below with reference to FIG. 12.

As illustrated in FIG. 12, when the contrast value has been input from the contrast value calculation section 336, the lens position control section 320 determines whether or not the input contrast value is larger than a threshold value Tc (S101). When the contrast value is larger than the threshold value Tc, the lens position control section 320 does not change the position of the focus lens 240 (S102). When the contrast value is equal to or smaller than the threshold value Tc, the lens position control section 320 compares the aperture value L of the light source aperture with a threshold value Tl (S103).

When the aperture value L of the light source aperture is smaller than the threshold value Tl, the lens position control section 320 moves the position of the focus lens 240 to the point B (i.e., the position that corresponds to the near point-side in-focus object plane position FPB in FIG. 4) (S104). When the aperture value L of the light source aperture is equal to or larger than the threshold value Tl, the lens position control section 320 moves the position of the focus lens 240 to the point A (i.e., the position that corresponds to the far point-side in-focus object plane position FPA in FIG. 4) (S105). The lens position control section 320 transmits the control signal that represents the position of the focus lens 240 to the image processing section 330, and terminates the process.

2.5.2 Screen display method

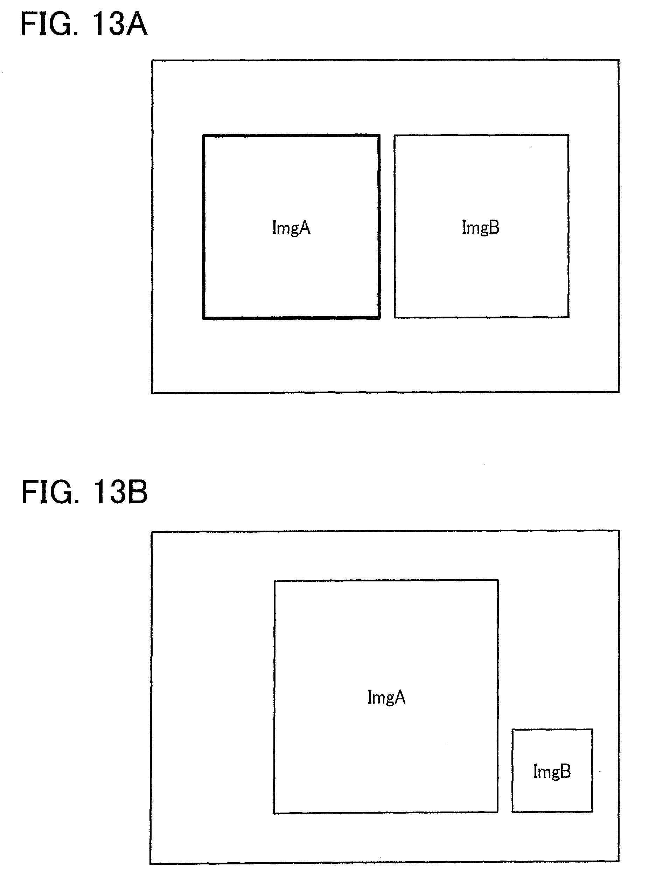

As illustrated in FIGS. 13A and 13B, two images may be displayed on the display section 400 (modification).

In this case, the freeze image setting section 337 extracts the captured image having the largest contrast value from the captured images stored in the storage section 334 that were captured when the lens position was set to the point A as a far-point freeze image, and extracts the captured image having the largest contrast value from the captured images stored in the storage section 334 that were captured when the lens position was set to the point B as a near-point freeze image. The freeze image setting section 337 transmits the near-point freeze image and the far-point freeze image to the selection section 333, and the selection section 333 transmits the near-point freeze image and the far-point freeze image to the post-processing section 338 when the freeze instruction signal has been input. The post-processing section 338 performs post-processing on the near-point freeze image and the far-point freeze image, and transmits the resulting near-point freeze image and the resulting far-point freeze image to the display section 400. The display section 400 displays the near-point freeze image and the far-point freeze image at the same time.

For example, a far-point freeze image ImgA and a near-point freeze image ImgB may be displayed to have the same size (see FIG. 13A), or may be displayed so that either the far-point freeze image ImgA or the near-point freeze image ImgB has a larger size (see FIG. 13B). The far-point freeze image ImgA or the near-point freeze image ImgB that was captured at the same lens position as the reference lens position may be displayed to have a larger size, or may be enclosed with a red frame or the like for enhancement. The user may select the storage target image from the far-point freeze image ImgA and the near-point freeze image ImgB through the external I/F section 500. In this case, the selected image is stored in a storage section (e.g., internal storage device or external storage device) (not illustrated in the drawings).

This makes it possible for the user to store one (or both) of the image captured at the near point-side in-focus object plane position and the image captured at the far point-side in-focus object plane position that is more suitable for observation.

Although an example in which the near-point freeze image and the far-point freeze image are extracted from the captured images stored in the storage section 334 has been described above, the configuration is not limited thereto. For example, when only the captured images captured at the near point-side in-focus object plane position FPB are stored in the storage section 334, the captured image may be acquired after moving the lens position to the point (far point) A to generate the far-point freeze image. When only the captured images captured at the far point A are stored in the storage section 334, the captured image may be acquired after moving the lens position to the point (near point) B to generate the near-point freeze image.

According to the first embodiment, the endoscope apparatus includes an image acquisition section (e.g., A/D conversion section 310 and demosaicing section 332), an in-focus evaluation value calculation section (contrast value calculation section 336), a focus control section (lens position control section 320), and the freeze image setting section 337 (see FIGS. 1 and 7). The image acquisition section acquires a plurality of *in vivo* images (i.e., captured images Img1 to ImgN in FIG. 9 (video in a narrow sense)) that include an image of *an in vivo* object and were obtained by capturing the *in vivo* object using the imaging optical system (objective lens 230, focus lens 240, and image sensor 260). The in-focus evaluation value calculation section calculates the in-focus evaluation value (contrast value Wc(t) in a narrow sense) that represents the degree of in-focus corresponding to each of the plurality of *in vivo* images. The focus control section controls the focus operation of the imaging optical system based on the in-focus evaluation value. The freeze image setting section 337 selects at least one *in vivo* image from the plurality of *in vivo* images based on the degree of in-focus represented by the in-focus evaluation value, and sets the selected at least one *in vivo* image to be the freeze image.

According to this configuration, since an image among the plurality of *in vivo* images that has a high degree of in-focus can be set to be the freeze image, it is possible to display the freeze image in which the object is accurately in focus even when the depth of field is shallow due to an increase in the number of pixels. Since the focus control section performs the AF control process, it is possible to display the freeze image in which the object is accurately in focus as compared with the case of manually bringing the object into focus.

The term "freeze image" used herein refers to a still image that is acquired when observing a video, and displayed or recorded. For example, the freeze image is acquired when the doctor desires to stop and closely observe a video, or when the doctor desires to take a second look after performing an endoscopic examination, or when the doctor desires to record a diseased part as an image.

The term "in-focus evaluation value" used herein refers to a value or information that is used to evaluate the degree of in-focus of the object within the captured image. For example, the contrast value is used as the in-focus evaluation value when using a contrast AF process. The contrast value is calculated by extracting a high-frequency component of the image, for example. Note that the in-focus evaluation value is not limited to the contrast value. Specifically, it suffices that the in-focus evaluation value be an evaluation value that becomes a maximum at the position of the object plane when the image plane coincides with the image plane of the image sensor, and decreases as the distance from the position of the object plane increases.

According to the first embodiment, the focus control section (lens position control section 320) controls the focus operation by performing a control process that switches the position of the focus adjustment lens (focus lens 240) included in the imaging optical system between a plurality of discrete positions (e.g., the positions A and B in FIG. 4) based on the in-focus evaluation value.

This makes it possible to simplify the AF control process as compared with the case of performing a continuous AF process. On the other hand, since the in-focus object plane position can only be set to a discrete position, the attention area that is the observation target of the doctor is not necessarily always in an ideal in-focus state, and there is a possibility that the freeze image in a good in-focus state cannot be captured when the freeze switch has been pressed. According to the first embodiment, however, since an image among the plurality of *in*

*vivo* images that has a high degree of in-focus can be set to be the freeze image, it is possible to display the freeze image in a better in-focus state even though the in-focus object plane position can only be set to a discrete position.

As described above with reference to FIG. 9, the captured image that was captured at the lens position A (far point) and the captured image that was captured at the lens position B (near point) are stored in the storage section 334. Since the depth of field is deep at the far point as compared with the near point, the contrast value tends to increase at the far point. When the captured image having a large contrast value is set to be the freeze image, the captured image that was captured at the far point tends to be set to be the freeze image. Therefore, when the attention area is situated at the near point, it is likely that the captured image in which an area (far point) other than the attention area is in focus is set to be the freeze image.

According to the first embodiment, the freeze image setting section 337 selects the freeze image from *in vivo* images among the plurality of *in vivo* images Img1 to ImgN that were captured at the same position as the position (position A in FIG. 9) of the focus adjustment lens used when an operation that instructs to acquire the freeze image was performed using the operation section (i.e., the freeze switch 270 or the freeze button provided to the external I/F section 500), as described above with reference to FIG. 9.

This makes it possible to accurately set the captured image in which the attention area is in focus to be the freeze image. Specifically, since it is considered that the freeze switch 270 is pressed when the doctor has determined that the attention area is in focus, the captured image in which an area other than the attention area is in focus can be excluded by selecting the freeze image from the images that were captured at the same lens position as the lens position used when the freeze instruction signal was input. Since the freeze image is selected from the near-point captured images when the attention area is situated at the near point, it is possible to prevent a situation in which the captured image in which an area (far point) other than the attention area is in focus is set to be the freeze image.

According to the first embodiment, the freeze image setting section 337 detects a blur state of each of the plurality of *in vivo* images based on the plurality of *in vivo* images, and selects the freeze image based on the blur state and the degree of in-focus, as described above with reference to FIG. 10, for example.

More specifically, the freeze image setting section 337 detects the motion amount $Wb(t)$ of the image of the *in vivo* object as the blur state, calculates the selection evaluation value $Fcb(t)$ by adding up a value obtained by multiplying the in-focus evaluation value $Wc(t)$ by a positive weight (coefficient a) and a value obtained by multiplying the motion amount $Wb(t)$ by a negative weight (coefficient b), and selects an *in vivo* image among the plurality of *in vivo* images Img1 to ImgN that has the largest selection evaluation value $Fcb(t)$ as the freeze image, as described above using the expression (1).

This makes it possible to suppress a situation in which the freeze image is blurred. Specifically, the captured image among the plurality of *in vivo* images in which the object is in focus and a motion blur is small can be displayed as the freeze image.

According to the first embodiment, the freeze image setting section 337 detects the elapsed time until each of the plurality of *in vivo* images was captured after an operation that instructs to acquire the freeze image was performed using the operation section (i.e., the freeze switch 270 or the freeze button provided to the external I/F section 500), and selects the freeze image based on the elapsed time and the degree of in-focus, as described above with reference to FIG. 11, for example.

More specifically, the freeze image setting section 337 calculates the elapsed time information $Wt(t)$ that increases in value as the elapsed time decreases, calculates the selection evaluation value $Fct(t)$ by adding up a value obtained by multiplying the in-focus evaluation value $Wc(t)$ by a given weight (coefficient c) and a value obtained by multiplying the elapsed time information $Wt(t)$ by a given weight (coefficient d), and selects an *in vivo* image among the plurality of *in vivo* images Img1 to ImgN that has the largest selection evaluation value $Fct(t)$ as the freeze image.

According to this configuration, the captured image among the plurality of *in vivo* images in which the object is in focus and which was captured at a timing closer to the freeze timing instructed by the user can be displayed as the freeze image. It is considered that the imaging range moves with the passing of time after the doctor has operated the freeze switch 270. According to the first embodiment, it is possible to display the freeze image that is close in imaging range to that when the freeze switch 270 was operated.

According to the first embodiment, the focus control section (lens position control section 320) performs a control process that switches the position of the focus adjustment lens (focus lens 240) between two discrete positions A and B that are used as the plurality of discrete positions.

More specifically, the focus control section determines whether or not the in-focus evaluation value is larger than the given threshold value Tc (S101), and maintains the current position of the focus adjustment lens (i.e., does not switch the position of the focus adjustment lens) when the focus control section has determined that the in-focus evaluation value is larger than the given threshold value (S102).

The endoscope apparatus includes the control section 340 that controls the intensity of the illumination light that illuminates the *in vivo* object, and outputs light intensity information (e.g., the opening of the aperture) that represents the light intensity L to the focus control section. The focus control section determines whether or not the light intensity L represented by the light intensity information is smaller than a given value TI when the focus control section has determined that the in-focus evaluation value is smaller than the given threshold value Tc (S103), switches the position of the focus adjustment lens to the near point-side position B included in the two discrete positions when the focus control section has determined that the light intensity L is smaller than the given value TI (S104), and switches the position of the focus adjustment lens to the far point-side position A included in the two discrete positions when the focus control section has determined that the light intensity L is larger than the given value TI.

This makes it possible to implement the AF process using a dual focus switch process, and simplify the AF control process. Since the position of the focus adjustment lens is not switched until it is determined that the in-focus evaluation value is smaller than the given threshold value, the in-focus object plane position is not frequently switched, and it is possible to provide the doctor with an image that is easy to observe. It is also possible to bring an object for which it is difficult to apply a contrast AF process (e.g., an object having low contrast) into focus by switching the lens position based on the light intensity L.

According to the first embodiment, the endoscope apparatus includes the attention area setting section 335 that sets the attention area to each of the plurality of *in vivo* images (see FIG. 7). The in-focus evaluation value calculation section calculates the in-focus evaluation value within the attention area. The freeze image setting section 337 selects the freeze image based on the degree of in-focus within the attention area that is represented by the in-focus evaluation value.

This makes it possible to set the captured image in which the area to which the user pays attention is in focus to be the freeze image. For example, since it is considered that the area to which the scope is brought closer is the observation target area of the doctor, a relatively bright area may be set to be the attention area (as described above with reference to FIG. 8).

The term "attention area" used herein refers to an area for which the observation priority for the user is relatively higher than that of other areas. For example, when the user is a doctor, and desires to perform treatment, the attention area refers to an area that includes a mucosal area or a lesion area. If the doctor desires to observe bubbles or feces, the attention area refers to an area that includes a bubble area or a feces area. Specifically, the attention area for the user differs depending on the object of observation, but necessarily has a relatively high observation priority as compared with the other areas.

According to the first embodiment, the endoscope apparatus includes the display section 400 that displays the freeze image (see FIG. 1). The focus control section continues to control the focus operation when the freeze image is displayed on the display section 400.

According to this configuration, since the focus operation can be performed while the freeze image is displayed, it is possible to capture an image in which the object is in focus when the freeze instruction signal has been canceled.

According to the first embodiment, the endoscope apparatus includes the selection section 333 (see FIG. 7). The selection section 333 receives the freeze image from the freeze image setting section 337 and the plurality of *in vivo* images from the image acquisition section, and selects the freeze image or the plurality of *in vivo* images as an image that is displayed on the display section 400.

According to this configuration, the endoscope apparatus can continue the AF control process that utilizes the captured image even when the freeze image is displayed while the captured image is not selected by the selection section 333. Specifically, when the freeze instruction signal has been input, transmission of the captured image from the image acquisition section (demosaicing section 332) to the storage section 334 is stopped, while the captured image is continuously transmitted from the image acquisition section to the attention area setting section 335. Therefore, the in-focus evaluation value calculation section (contrast value calculation section 336) can calculate the in-focus evaluation value of the attention area, and the lens position control section 320 can perform the focus operation based on the in-focus evaluation value.

3. Second embodiment (continuous AF)

3.1. Endoscope apparatus

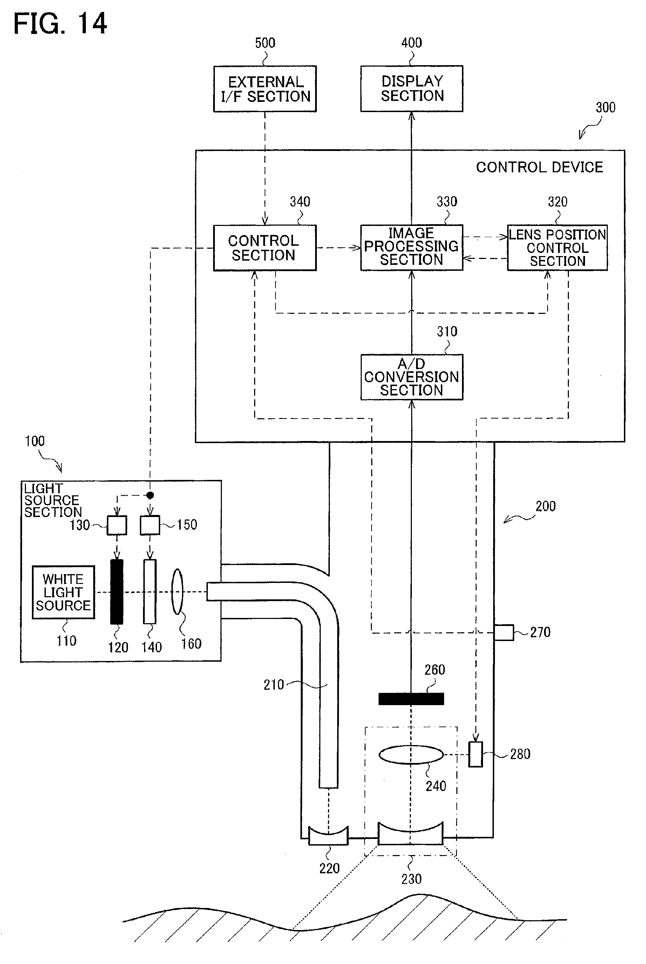

FIG. 14 illustrates a configuration example of an endoscope apparatus according to the second embodiment. The endoscope apparatus includes a light source section 100, an imaging section 200, a control device 300 (processor section), a display section 400, and an external I/F section 500. Note that the same elements as those described above in connection with the first embodiment are respectively indicated by the same reference signs (symbols), and description thereof is appropriately omitted.

The imaging section 200 includes a light guide fiber 210, an illumination lens 220, an objective lens 230, a focus lens 240, an image sensor 260, a freeze switch 270, and a lens driver section 280. The lens driver section 280 continuously drives the position of the focus lens 240 based on an instruction issued by a lens position control section 320 (i.e., continuous AF process).

The term "continuous AF process" used herein refers to an AF process that continuously performs an operation that brings the object into focus. More specifically, the continuous AF process wobbles the focus lens 240 to determine the in-focus lens position, and then wobbles the focus lens 240 using the determined lens position as a reference. This operation is repeated during the continuous AF process. In this case, the focus lens 240 can be moved to an arbitrary (e.g., non-discrete) position within a given position range (e.g., the range from the position A to the position B in FIG. 4).

3.2. Continuous AF operation

The continuous AF operation that is implemented according to the second embodiment is described in detail below. The round-trip width of the focus lens 240 during wobbling is referred to as ±dw, and the moving width (focal distance update value) of the focus lens 240 up to the lens position determined by wobbling is referred to as dn.

The lens position control section 320 changes the position of the focus lens 240 to a position ds-dw through the lens driver section 280, and stores information about the position ds-dw of the focus lens 240. The position ds is the initial position (reference position) of the focus lens 240 during wobbling. The contrast value calculation section 336 calculates a contrast value C(-dw) at the position ds-dw, and transmits the calculated contrast value C(-dw) to the lens position control section 320. The lens position control section 320 changes the position of the focus lens 240 to the position ds+dw through the lens driver section 280, and stores information about the position ds+dw of the focus lens 240. The contrast value calculation section 336 calculates a contrast value C(+dw) at the position ds+dw, and transmits the calculated contrast value C(+dw) to the lens position control section 320.

The lens position control section 320 then updates the initial position ds based on the position information about the focus lens 240 and the contrast value transmitted from the contrast value calculation section 336. More specifically, the lens position control section 320 decreases the value ds by the value dn (i.e., sets the position ds-dn to be the initial position ds) when C(-dw)>C(+dw), and increases the value ds by the value dn (i.e., sets the position ds+dn to be the initial position ds) when C(+dw)>C(-dw). The moving width dn of the focus lens 240 may be calculated using a hill-climbing method, for example. Specifically, the position of the focus lens 240 at which the contrast value becomes a maximum is estimated from the contrast values C(-dw), C(0), and C(+dw), and determined to be the moving width dn.

The lens position control section 320 transmits the lens position ds-dw obtained by subtracting the round-trip width dw during wobbling from the updated initial position ds of the focus lens 240 to the lens driver section 280. The above process is repeated.

Note that the continuous AF operation according to the second embodiment is not limited to the above operation. For example, the values dw and dn may be set to a constant value in advance, or the user may set the values dw and dn to an arbitrary value through the external I/F section 500. Although an example in which the round-trip width dw during wobbling is fixed has been described above, the configuration is not limited thereto. For example, the round-trip width dw may be increased when the freeze image is displayed as compared with the case where the freeze image is not displayed. According to this configuration, it is possible to implement a highly accurate focus operation that can follow a large motion of the object when the freeze image is displayed.

Note that the imaging optical system that is controlled by the lens position control section according to the second embodiment is an optical system that adjusts the focus while changing the angle of view (imaging magnification) by driving (operating) the zoom lens. Note that the configuration is not limited thereto. It is also possible to use an imaging optical system that can independently adjust the position of the zoom lens and the position of the focus lens.

3.3. Freeze image setting section

The operation of the freeze image setting section 337 is described in detail below. The freeze image setting section 337 according to the second embodiment differs from the freeze image setting section 337 according to the first embodiment as to the lens position. Specifically, while the focus lens 240 is set to a discrete position in the first embodiment, the focus lens 240 is set to a continuous position in the second embodiment.

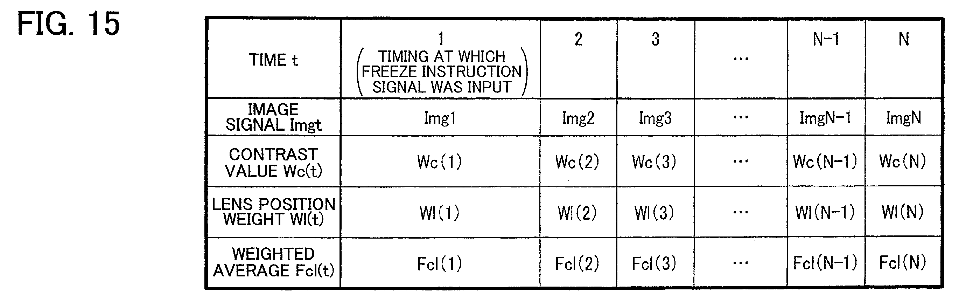

As illustrated in FIG. 15, the position of the focus lens 240 at a time t=1 is used as the reference lens position. The time t=1 is a timing at which the freeze switch 270 (or the freeze button) was operated, and the freeze instruction signal was input. The freeze image setting section 337 calculates a lens position weight Wl(t) that increases as the distance from the reference lens position decreases, and calculates a weighted average Fcl(t) of a contrast value Wc(t) and the lens position weight Wl(t) using the following expression (3). In this case, it is possible to set the captured image that has a large contrast value and was captured at the in-focus object plane position closer to that used at the timing at which the user performed the freeze operation to be the freeze image.

$$Fcl(t)=e{\times}Wc(t)+f{\times}Wl(t) \qquad (3)$$

Note that e is a constant that satisfies e≥0, and f is a constant that satisfies f≥0. A value input in advance from the outside, a value set in advance to the control section 340, or the like is used as the constants e and f. When the lens position at a time t is lp(t), the lens position weight Wl(t) is Wl(t)=-|lp(t)-lp(1)|.

According to the above configuration (continuous AF process), it is possible to implement a finer focus operation as compared with a focus-switch AF process, and accurately obtain a freeze image in which the object is in focus.

According to the second embodiment, the focus control section (lens position control section 320) controls the focus operation by performing a control process that moves the position of the focus adjustment lens (focus lens 240) included in the imaging optical system within a continuous position range based on the in-focus evaluation value. The freeze image setting section 337 acquires lens position information that represents the difference between the position (reference lens position) of the focus adjustment lens when an operation that instructs to acquire the freeze image was performed using the operation section (i.e., the freeze switch 270 or the freeze button provided to the external I/F section 500), and the position of the focus adjustment lens when each of the plurality of *in vivo* images was captured, and selects the freeze image based on the lens position information and the degree of in-focus, as described above with reference to FIG. 15.

More specifically, the freeze image setting section 337 acquires the lens position information WI(t) that increases in value as the difference between the position of the focus adjustment lens when an operation that instructs to acquire the freeze image was performed using the operation section, and the position of the focus adjustment lens when each of the plurality of *in vivo* images was captured, decreases, calculates the selection evaluation value Fcl(t) by adding up a value obtained by multiplying the in-focus evaluation value Wc(t) by a given weight (coefficient e) and a value obtained by multiplying the lens position information WI(t) by a given weight (coefficient f), and selects an *in vivo* image among the plurality of *in vivo* images Img1 to ImgN that has the largest selection evaluation value Fcl(t) as the freeze image.

According to this configuration, the captured image among the plurality of *in vivo* images in which the object is in focus and which was captured at a timing closer to the freeze timing instructed by the user can be displayed as the freeze image. It is considered that the imaging range moves with the passing of time, and the lens position moves through the contrast AF process after the doctor has operated the freeze switch 270. According to the second embodiment, it is possible to display a freeze image that is close in imaging range to that when the freeze switch 270 was operated.

According to the second embodiment, the endoscope apparatus includes the control section 340 that sets the imaging condition for the imaging optical system (see FIG. 14). The control section 340 changes the imaging condition between the case where a plurality of *in vivo* images (videos) are displayed on the display section 400 and the case where the freeze image is displayed on the display section 400.

More specifically, the imaging condition is the exposure time, or the wobbling width when the continuous AF process is performed as the focus operation. The control section 340 increases the exposure time or the wobbling width dw when the freeze image is displayed on the display section 400 as compared with the case where a plurality of *in vivo* images are displayed on the display section 400.

According to this configuration, it is possible to improve the capability to continuously bring the object into focus by changing the imaging condition while the freeze image is displayed. Since the user cannot observe an image captured under the imaging condition that has been changed while the freeze image is displayed, no problem occurs even if the imaging condition is changed while the freeze image is displayed.

Note that the term "imaging condition" used herein refers to a condition whereby the capability to bring the object into focus is improved during the focus operation. For example, the imaging condition is the exposure time or the wobbling width. Note that the configuration is not limited thereto. The imaging condition may be a frame rate or the like.

The embodiments to which the invention is applied and the modifications thereof have been described above. Note that the invention is not limited to the above embodiments and the modifications thereof. Various modifications and variations may be made of the above embodiments and the modifications thereof without departing from the scope of the invention. A plurality of elements described in connection with the above embodiments and the modifications thereof may be appropriately combined to implement various configurations. For example, some elements may be omitted from the elements described in connection with the above embodiments and the modifications thereof. Some of the elements described above in connection with different embodiments or modifications thereof may be appropriately combined. Accordingly, various modifications and applications are possible without materially departing from the novel teachings and advantages of the invention.

Any term (e.g., capsule endoscope, scope-type endoscope, and white light image) cited with a different term (e.g., first endoscope apparatus, second endoscope apparatus, and normal light image) having a broader meaning or the same meaning at least once in the specification and the drawings may be replaced by the different term in any place in the specification and the drawings.

REFERENCE SIGNS LIST

100: light source section, 110: white light source, 130: light source aperture driver section, 140: rotary color filter, 150: rotation section, 160: condenser lens, 200: imaging section, 210: light guide fiber, 220: illumination lens, 230: objective lens, 240: focus lens, 250: switch section, 260: image sensor, 270: freeze switch, 280: lens driver section, 300: control device, 310: A/D conversion section, 320: lens position control section, 330: image processing section, 331: pre-processing section, 332: demosaicing section, 333:

selection section, 334: storage section, 335: attention area setting section, 336: contrast value calculation section, 337: freeze image setting section, 338: post-processing section, 340: control section, 400: display section, 500: external I/F section, 701 to 704: filter, 704: rotary motor, A, B: lens position, BR1 to BR5: area, DFA, DFB: depth of field, FPA, FPB: in-focus object plane position, Fcb(t), Fcl(t), Fct(t): selection evaluation value, Imgt: captured image, ImgA: far-point freeze image, ImgB: near-point freeze image, L: light intensity, Tl: given value, Tc, Tl: threshold value, Wb(t): amount of blur, Wc(t): contrast value, Wl(t): lens position information, Wt(t): elapsed time information, a to f: coefficient, t: time

**Claims**

1. An endoscope apparatus comprising:

   an image acquisition section that acquires a plurality of *in vivo* images that were obtained by capturing an *in vivo* object using an imaging optical system, each of the plurality of *in vivo* images including an image of the *in vivo* object; an in-focus evaluation value calculation section that calculates an in-focus evaluation value that represents a degree of in-focus corresponding to each of the plurality of *in vivo* images; a focus control section that controls a focus operation of the imaging optical system based on the in-focus evaluation value; and a freeze image setting section that selects at least one *in vivo* image from the plurality of *in vivo* images based on the degree of in-focus represented by the in-focus evaluation value, and sets the selected at least one *in vivo* image to be a freeze image.

2. The endoscope apparatus as defined in claim 1, wherein the focus control section controls the focus operation by performing a control process that switches a position of a focus adjustment lens included in the imaging optical system between a plurality of discrete positions based on the in-focus evaluation value.

3. The endoscope apparatus as defined in claim 2, wherein the freeze image setting section selects the freeze image from *in vivo* images among the plurality of *in vivo* images that were captured at the same position as the position of the focus adjustment lens used when an operation that instructs to acquire the freeze image was performed using an operation section.

4. The endoscope apparatus as defined in claim 3, wherein the in-focus evaluation value calculation section calculates the in-focus evaluation value that increases as the degree of in-focus increases, and the freeze image setting section selects an *in vivo* image that has a largest in-focus evaluation value as the freeze image, the *in vivo* image that has the largest in-focus evaluation value being selected from the *in vivo* images among the plurality of *in vivo* images that were captured at the same position as the position of the focus adjustment lens used when the operation that instructs to acquire the freeze image was performed using the operation section.

5. The endoscope apparatus as defined in claim 3, further comprising:

   a storage section that stores the plurality of *in vivo* images, wherein the image acquisition section acquires first to Nth *in vivo* images (where, N is a natural number equal to or larger than 2) as the plurality of *in vivo* images, the storage section stores an ith *in vivo* image (where, i is a natural number that satisfies $1 \leq i \leq N$) among the first to Nth *in vivo* images, the in-focus evaluation value that corresponds to the ith *in vivo* image, and the position of the focus adjustment lens when the ith *in vivo* image was captured in a linked state, and the freeze image setting section selects the freeze image by referring to the storage section.

6. The endoscope apparatus as defined in claim 1, wherein the freeze image setting section detects a blur state of each of the plurality of *in vivo* images based on the plurality of *in vivo* images, and selects the freeze image based on the blur state and the degree of in-focus.

7. The endoscope apparatus as defined in claim 6, wherein the in-focus evaluation value calculation section calculates the in-focus evaluation value that increases as the degree of in-focus increases, and the freeze image setting section detects a motion amount of the image of the *in vivo* object as the blur state, calculates a selection evaluation value by adding up a value obtained by multiplying the in-focus evaluation value by a positive weight and a value obtained by multiplying the motion amount by a negative weight, and selects an *in vivo* image among the plurality of *in vivo* images that has a largest selection evaluation value as the freeze image.

8. The endoscope apparatus as defined in claim 7, wherein the focus control section controls the focus operation by performing a control process that switches a position of a focus adjustment lens included in the imaging optical system between a plurality of discrete positions based on the in-focus evaluation value, and

the freeze image setting section selects an *in vivo* image that has the largest selection evaluation value as the freeze image, the *in vivo* image that has the largest selection evaluation value being selected from *in vivo* images among the plurality of *in vivo* images that were captured at the same position as the position of the focus adjustment lens used when an operation that instructs to acquire the freeze image was performed using an operation section.

9. The endoscope apparatus as defined in claim 1, wherein the freeze image setting section detects an elapsed time until each of the plurality of *in vivo* images was captured after an operation that instructs to acquire the freeze image was performed using an operation section, and selects the freeze image based on the elapsed time and the degree of in-focus.

10. The endoscope apparatus as defined in claim 9, wherein the in-focus evaluation value calculation section calculates the in-focus evaluation value that increases as the degree of in-focus increases, and the freeze image setting section calculates elapsed time information that increases in value as the elapsed time decreases, calculates a selection evaluation value by adding up a value obtained by multiplying the in-focus evaluation value by a given weight and a value obtained by multiplying the elapsed time information by a given weight, and selects an *in vivo* image among the plurality of *in vivo* images that has a largest selection evaluation value as the freeze image.

11. The endoscope apparatus as defined in claim 10, wherein the focus control section controls the focus operation by performing a control process that switches a position of a focus adjustment lens included in the imaging optical system between a plurality of discrete positions based on the in-focus evaluation value, and

the freeze image setting section selects an *in vivo* image that has the largest selection evaluation value as the freeze image, the *in vivo* image that has the largest selection evaluation value being selected from *in vivo* images among the plurality of *in vivo* images that were captured at the same position as the position of the focus adjustment lens used when an operation that instructs to acquire the freeze image was performed using the operation section.

12. The endoscope apparatus as defined in claim 2, wherein the focus control section performs a control process that switches the position of the focus adjustment lens between two discrete positions that are used as the plurality of discrete positions.

13. The endoscope apparatus as defined in claim 12, wherein the in-focus evaluation value calculation section calculates the in-focus evaluation value that increases as the degree of in-focus increases, and the focus control section determines whether or not the in-focus evaluation value is larger than a given threshold value, and maintains a current position of the focus adjustment lens when the focus control section has determined that the in-focus evaluation value is larger than the given threshold value.

14. The endoscope apparatus as defined in claim 13, further comprising:

a control section that controls a light intensity of illumination light that illuminates the *in vivo* object, and outputs light intensity information that represents the light intensity to the focus control section,

wherein the focus control section determines whether or not the light intensity represented by the light intensity information is smaller than a given value when the focus control section has determined that the in-focus evaluation value is smaller than the given threshold value, switches the position of the focus adjustment lens to a near point-side position included in the two discrete positions when the focus control section has determined that the light intensity is smaller than the given value, and switches the position of the focus adjustment lens to a far point-side position included in the two discrete positions when the focus control section has determined that the light intensity is larger than the given value.

15. The endoscope apparatus as defined in claim 1, further comprising:

an attention area setting section that sets an attention area to each of the plurality of *in vivo* images,

wherein the in-focus evaluation value calculation section calculates the in-focus evaluation value within the attention area, and

the freeze image setting section selects the freeze image based on the degree of in-focus within the attention area that is represented by the in-focus evaluation value.

16. The endoscope apparatus as defined in claim 1, further comprising:

a display section that displays the freeze image, wherein the focus control section continues to control the focus operation when the freeze image is displayed on the display section.

**17.** The endoscope apparatus as defined in claim 16, further comprising:

> a selection section that receives the freeze image from the freeze image setting section and the plurality of *in vivo* images from the image acquisition section, and selects the freeze image or the plurality of *in vivo* images as an image that is displayed on the display section.

**18.** The endoscope apparatus as defined in claim 16, further comprising:

> a control section that sets an imaging condition for the imaging optical system,
> wherein the control section changes the imaging condition between a case where the plurality of *in vivo* images are displayed on the display section and a case where the freeze image is displayed on the display section.

**19.** The endoscope apparatus as defined in claim 18, wherein the imaging condition is an exposure time, or a wobbling width when a continuous AF process is performed as the focus operation, and the control section increases the exposure time or the wobbling width when the freeze image is displayed on the display section as compared with a case where the plurality of *in vivo* images are displayed on the display section.

**20.** The endoscope apparatus as defined in claim 1, further comprising:

> a display section that displays the freeze image, wherein the freeze image setting section selects two or more *in vivo* images as the at least one *in vivo* image that is set to be the freeze image, the display section displays the two or more *in vivo* images selected as the freeze image, and the freeze image setting section sets an *in vivo* image selected by a user from the two or more *in vivo* images displayed on the display section through an operation section to be an *in vivo* image that is stored in a storage section.

**21.** The endoscope apparatus as defined in claim 1, wherein the focus control section controls the focus operation by performing a control process that switches a position of a focus adjustment lens included in the imaging optical system within a continuous position range based on the in-focus evaluation value, and the freeze image setting section acquires lens position information, and selects the freeze image based on the lens position information and the degree of in-focus, the lens position information representing a difference between the position of the focus adjustment lens when an operation that instructs to acquire the freeze image was performed using an operation section, and the position of the focus adjustment lens when each of the plurality of *in vivo* images was captured.

**22.** The endoscope apparatus as defined in claim 21, wherein the freeze image setting section acquires the lens position information that increases in value as the difference between the position of the focus adjustment lens when the operation that instructs to acquire the freeze image was performed using the operation section, and the position of the focus adjustment lens when each of the plurality of *in vivo* images was captured, decreases, calculates a selection evaluation value by adding up a value obtained by multiplying the in-focus evaluation value by a given weight and a value obtained by multiplying the lens position information by a given weight, and selects an *in vivo* image among the plurality of *in vivo* images that has a largest selection evaluation value as the freeze image.

**23.** A method for controlling an endoscope apparatus comprising:

> acquiring a plurality of *in vivo* images that were obtained by capturing an *in vivo* object using an imaging optical system, each of the plurality of *in vivo* images including an image of the *in vivo* object;
> calculating an in-focus evaluation value that represents a degree of in-focus corresponding to each of the plurality of *in vivo* images;
> controlling a focus operation of the imaging optical system based on the in-focus evaluation value; and
> selecting at least one *in vivo* image from the plurality of *in vivo* images based on the degree of in-focus represented by the in-focus evaluation value, and setting the selected at least one *in vivo* image to be a freeze image.

**Amended claims under Art. 19.1 PCT**

**1.** (Amended) An endoscope apparatus comprising:

> an image acquisition section that acquires a plurality of *in vivo* images that were obtained by capturing an *in vivo* object using an imaging optical system, each of the plurality of *in vivo* images including an image of the *in vivo* object;
> an in-focus evaluation value calculation section that calculates an in-focus evaluation value that represents a degree of in-focus corresponding to each of the plurality of *in vivo* images;
> a focus control section that controls a focus operation of the imaging optical system by performing a control process that switches a position of a focus adjustment lens included in the imaging optical system between a plurality of discrete positions based on the in-focus evaluation value; and

a freeze image setting section that selects at least one *in vivo* image from the plurality of *in vivo* images based on the degree of in-focus represented by the in-focus evaluation value, and sets the selected at least one *in vivo* image to be a freeze image.

2. (Deleted)

3. (Amended) The endoscope apparatus as defined in claim 1,
wherein the freeze image setting section selects the freeze image from *in vivo* images among the plurality of *in vivo* images that were captured at the same position as the position of the focus adjustment lens used when an operation that instructs to acquire the freeze image was performed using an operation section.

4. The endoscope apparatus as defined in claim 3,
wherein the in-focus evaluation value calculation section calculates the in-focus evaluation value that increases as the degree of in-focus increases, and the freeze image setting section selects an *in vivo* image that has a largest in-focus evaluation value as the freeze image, the *in vivo* image that has the largest in-focus evaluation value being selected from the *in vivo* images among the plurality of *in vivo* images that were captured at the same position as the position of the focus adjustment lens used when the operation that instructs to acquire the freeze image was performed using the operation section.

5. The endoscope apparatus as defined in claim 3, further comprising:

a storage section that stores the plurality of *in vivo* images,
wherein the image acquisition section acquires first to Nth *in vivo* images (where, N is a natural number equal to or larger than 2) as the plurality of *in vivo* images,
the storage section stores an ith *in vivo* image (where, i is a natural number that satisfies $1 \leq i \leq N$) among the first to Nth *in vivo* images, the in-focus evaluation value that corresponds to the ith *in vivo* image, and the position of the focus adjustment lens when the ith *in vivo* image was captured in a linked state, and
the freeze image setting section selects the freeze image by referring to the storage section.

6. The endoscope apparatus as defined in claim 1,
wherein the freeze image setting section detects a blur state of each of the plurality of *in vivo* images based on the plurality of *in vivo* images, and selects the freeze image based on the blur state and the degree of in-focus.

7. The endoscope apparatus as defined in claim 6,
wherein the in-focus evaluation value calculation section calculates the in-focus evaluation value that increases as the degree of in-focus increases, and the freeze image setting section detects a motion amount of the image of the *in vivo* object as the blur state, calculates a selection evaluation value by adding up a value obtained by multiplying the in-focus evaluation value by a positive weight and a value obtained by multiplying the motion amount by a negative weight, and selects an *in vivo* image among the plurality of *in vivo* images that has a largest selection evaluation value as the freeze image.

8. (Amended) The endoscope apparatus as defined in claim 7,
the freeze image setting section selects an *in vivo* image that has the largest selection evaluation value as the freeze image, the *in vivo* image that has the largest selection evaluation value being selected from *in vivo* images among the plurality of *in vivo* images that were captured at the same position as the position of the focus adjustment lens used when an operation that instructs to acquire the freeze image was performed using an operation section.

9. The endoscope apparatus as defined in claim 1,
wherein the freeze image setting section detects an elapsed time until each of the plurality of *in vivo* images was captured after an operation that instructs to acquire the freeze image was performed using an operation section, and selects the freeze image based on the elapsed time and the degree of in-focus.

10. The endoscope apparatus as defined in claim 9,
wherein the in-focus evaluation value calculation section calculates the in-focus evaluation value that increases as the degree of in-focus increases, and the freeze image setting section calculates elapsed time information that increases in value as the elapsed time decreases, calculates a selection evaluation value by adding up a value obtained by multiplying the in-focus evaluation value by a given weight and a value obtained by multiplying the elapsed time information by a given weight, and selects an *in vivo* image among the plurality of *in vivo* images that has a largest selection evaluation value as the freeze image.

11. (Amended) The endoscope apparatus as defined in claim 10,

the freeze image setting section selects an *in vivo* image that has the largest selection evaluation value as the freeze image, the *in vivo* image that has the largest selection evaluation value being selected from *in vivo* images among the plurality of *in vivo* images that were captured at the same position as the position of the focus adjustment lens used when an operation that instructs to acquire the freeze image was performed using the operation section.

**12.** (Amended) The endoscope apparatus as defined in claim 1,

wherein the focus control section performs a control process that switches the position of the focus adjustment lens between two discrete positions that are used as the plurality of discrete positions.

**13.** The endoscope apparatus as defined in claim 12, wherein the in-focus evaluation value calculation section calculates the in-focus evaluation value that increases as the degree of in-focus increases, and the focus control section determines whether or not the in-focus evaluation value is larger than a given threshold value, and maintains a current position of the focus adjustment lens when the focus control section has determined that the in-focus evaluation value is larger than the given threshold value.

**14.** The endoscope apparatus as defined in claim 13, further comprising:

a control section that controls a light intensity of illumination light that illuminates the *in vivo* object, and outputs light intensity information that represents the light intensity to the focus control section,

wherein the focus control section determines whether or not the light intensity represented by the light intensity information is smaller than a given value when the focus control section has determined that the in-focus evaluation value is smaller than the given threshold value, switches the position of the focus adjustment lens to a near point-side position included in the two discrete positions when the focus control section has determined that the light intensity is smaller than the given value, and switches the position of the focus adjustment lens to a far point-side position included in the two discrete positions when the focus control section has determined that the light intensity is larger than the given value.

**15.** The endoscope apparatus as defined in claim 1, further comprising:

an attention area setting section that sets an attention area to each of the plurality of *in vivo* images,

wherein the in-focus evaluation value calculation section calculates the in-focus evaluation value within the attention area, and

the freeze image setting section selects the freeze image based on the degree of in-focus within the attention area that is represented by the in-focus evaluation value.

**16.** The endoscope apparatus as defined in claim 1, further comprising:

a display section that displays the freeze image, wherein the focus control section continues to control the focus operation when the freeze image is displayed on the display section.

**17.** The endoscope apparatus as defined in claim 16, further comprising:

a selection section that receives the freeze image from the freeze image setting section and the plurality of *in vivo* images from the image acquisition section, and selects the freeze image or the plurality of *in vivo* images as an image that is displayed on the display section.

**18.** The endoscope apparatus as defined in claim 16, further comprising:

a control section that sets an imaging condition for the imaging optical system,

wherein the control section changes the imaging condition between a case where the plurality of *in vivo* images are displayed on the display section and a case where the freeze image is displayed on the display section.

**19.** The endoscope apparatus as defined in claim 18, wherein the imaging condition is an exposure time, or a wobbling width when a continuous AF process is performed as the focus operation, and the control section increases the exposure time or the wobbling width when the freeze image is displayed on the display section as compared with a case where the plurality of *in vivo* images are displayed on the display section.

**20.** The endoscope apparatus as defined in claim 1, further comprising:

a display section that displays the freeze image, wherein the freeze image setting section selects two or more *in vivo* images as the at least one *in vivo* image that is set to be the freeze image, the display section displays the two or more *in vivo* images selected as the freeze image, and

the freeze image setting section sets an *in vivo* image selected by a user from the two or more *in vivo* images displayed on the display section through an operation section to be an *in vivo* image that is stored in a storage section.

21. (Amended) The endoscope apparatus as defined in claim 1,
the freeze image setting section acquires lens position information, and selects the freeze image based on the lens position information and the degree of in-focus, the lens position information representing a difference between the position of the focus adjustment lens when an operation that instructs to acquire the freeze image was performed using an operation section, and the position of the focus adjustment lens when each of the plurality of *in vivo* images was captured.

22. The endoscope apparatus as defined in claim 21, wherein the freeze image setting section acquires the lens position information that increases in value as the difference between the position of the focus adjustment lens when the operation that instructs to acquire the freeze image was performed using the operation section, and the position of the focus adjustment lens when each of the plurality of *in vivo* images was captured, decreases, calculates a selection evaluation value by adding up a value obtained by multiplying the in-focus evaluation value by a given weight and a value obtained by multiplying the lens position information by a given weight, and selects an *in vivo* image among the plurality of *in vivo* images that has a largest selection evaluation value as the freeze image.

23. (Amended) A method for controlling an endoscope apparatus comprising:

acquiring a plurality of *in vivo* images that were obtained by capturing an *in vivo* object using an imaging optical system, each of the plurality of *in vivo* images including an image of the *in vivo* object;
calculating an in-focus evaluation value that represents a degree of in-focus corresponding to each of the plurality of *in vivo* images;
controlling a focus operation of the imaging optical system by performing a control process that switches a position of a focus adjustment lens included in the imaging optical system between a plurality of discrete positions based on the in-focus evaluation value; and
selecting at least one *in vivo* image from the plurality of *in vivo* images based on the degree of in-focus represented by the in-focus evaluation value, and setting the selected at least one *in vivo* image to be a freeze image.

FIG. 1

# FIG. 2

# FIG. 3

# FIG. 4

DFA
(DEPTH OF FIELD)

DFB
(DEPTH OF FIELD)

FPA
(IN-FOCUS OBJECT
PLANE POSITION)

FPB
(IN-FOCUS OBJECT
PLANE POSITION)

DISTANCE FROM
IMAGING SECTION

A

B

FAR POINT SIDE

FOCUS LENS
POSITION

NEAR POINT SIDE

22

# FIG. 5

OBJECT
(INTERNAL ORGAN)

DEPTH OF FIELD

IMAGING SECTION

IN-FOCUS OBJECT
PLANE POSITION

# FIG. 6

DEPTH OF FIELD

OBJECT
(INTERNAL ORGAN)

IMAGING SECTION

IN-FOCUS OBJECT
PLANE POSITION

## FIG. 7

EP 3 050 484 A1

# FIG. 8

BR2   BR3   BR1   BR4   BR5

FIG. 9

| TIME t | 1 (TIMING AT WHICH FREEZE INSTRUCTION SIGNAL WAS INPUT) | 2 | 3 | ... | N-1 | N |
|---|---|---|---|---|---|---|
| CAPTURED IMAGE Imgt | Img1 | Img2 | Img3 | ... | ImgN-1 | ImgN |
| CONTRAST VALUE Wc(t) | Wc(1) | Wc(2) | Wc(3) | ... | Wc(N-1) | Wc(N) |
| LENS POSITION | A | A | B | ... | B | A |

FIG. 10

| TIME t | 1 (TIMING AT WHICH FREEZE INSTRUCTION SIGNAL WAS INPUT) | 2 | 3 | ... | N-1 | N |
|---|---|---|---|---|---|---|
| IMAGE SIGNAL Imgt | Img1 | Img2 | Img3 | ... | ImgN-1 | ImgN |
| CONTRAST VALUE Wc(t) | Wc(1) | Wc(2) | Wc(3) | ... | Wc(N-1) | Wc(N) |
| MOTION BLUR Wb(t) | Wb(1) | Wb(2) | Wb(3) | ... | Wb(N-1) | Wb(N) |
| WEIGHTED AVERAGE Fcb(t) | Fcb(1) | Fcb(2) | Fcb(3) | ... | Fcb(N-1) | Fcb(N) |
| LENS POSITION | A | A | B | ... | B | A |

27

## FIG. 11

| TIME t | 1<br>(TIMING AT WHICH<br>FREEZE INSTRUCTION<br>SIGNAL WAS INPUT) | 2 | 3 | ... | N-1 | N |
|---|---|---|---|---|---|---|
| IMAGE<br>SIGNAL Imgt | Img1 | Img2 | Img3 | ... | ImgN-1 | ImgN |
| CONTRAST<br>VALUE Wc(t) | Wc(1) | Wc(2) | Wc(3) | ... | Wc(N-1) | Wc(N) |
| TIME WEIGHT<br>Wt(t) | Wt(1) | Wt(2) | Wt(3) | ... | Wt(N-1) | Wt(N) |
| WEIGHTED<br>AVERAGE Fcb(t) | Fct(1) | Fct(2) | Fct(3) | ... | Fct(N-1) | Fct(N) |
| LENS POSITION | A | A | B | ... | B | A |

EP 3 050 484 A1

# FIG. 12

START

S101

CONTRAST
VALUE>Tc?

NO ← → YES

S103

L>Tl?

NO ← → YES

| MOVE FOCUS LENS<br>TO POINT A | MOVE FOCUS LENS<br>TO POINT B | DO NOT CHANGE FOCUS<br>LENS POSITION |
|---|---|---|
| S105 | S104 | S102 |

END

EP 3 050 484 A1

FIG. 13A

FIG. 13B

# FIG. 14

**FIG. 15**

| TIME t | 1 (TIMING AT WHICH FREEZE INSTRUCTION SIGNAL WAS INPUT) | 2 | 3 | ... | N−1 | N |
|---|---|---|---|---|---|---|
| IMAGE SIGNAL Imgt | Img1 | Img2 | Img3 | ... | ImgN−1 | ImgN |
| CONTRAST VALUE Wc(t) | Wc(1) | Wc(2) | Wc(3) | ... | Wc(N−1) | Wc(N) |
| LENS POSITION WEIGHT Wl(t) | Wl(1) | Wl(2) | Wl(3) | ... | Wl(N−1) | Wl(N) |
| WEIGHTED AVERAGE Fcl(t) | Fcl(1) | Fcl(2) | Fcl(3) | ... | Fcl(N−1) | Fcl(N) |

**INTERNATIONAL SEARCH REPORT**

International application No.

PCT/JP2013/075625

A. CLASSIFICATION OF SUBJECT MATTER
*A61B1/00*(2006.01)i

According to International Patent Classification (IPC) or to both national classification and IPC

B. FIELDS SEARCHED

Minimum documentation searched (classification system followed by classification symbols)
A61B1/00-1/32, G02B23/24-23/26

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2010-220755 A (Fujifilm Corp.),<br>07 October 2010 (07.10.2010),<br>entire text; fig. 1 to 11<br>entire text; fig. 1 to 11<br>(Family: none) | 1,6-8,15-17<br>2-5,9-14,<br>18-22 |
| Y | JP 2003-32521 A (Nikon Corp.),<br>31 January 2003 (31.01.2003),<br>entire text; fig. 1 to 6<br>(Family: none) | 1,6-8,15-17 |
| Y | JP 2010-152361 A (Mitsutoyo Corp.),<br>08 July 2010 (08.07.2010),<br>entire text; fig. 1 to 10<br>& US 2010/0158343 A1 & DE 102009029478 A | 15 |

| ☒ Further documents are listed in the continuation of Box C. | ☐ See patent family annex. |
|---|---|

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>17 December, 2013 (17.12.13) | Date of mailing of the international search report<br>07 January, 2014 (07.01.14) |
|---|---|
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/075625

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2005-102199 A  (Canon Inc.),<br>14 April 2005 (14.04.2005),<br>entire text; fig. 1 to 15<br>& US 2005/0128341 A1    & EP 1667433 A1<br>& WO 2005/025212 A1 | 1-22 |
| A | JP 2001-228412 A  (Fuji Photo Optical Co.,<br>Ltd.),<br>24 August 2001 (24.08.2001),<br>entire text; fig. 1 to 7<br>& US 2001/0016680 A1 | 1-22 |
| A | JP 10-179506 A  (Olympus Optical Co., Ltd.),<br>07 July 1998 (07.07.1998),<br>entire text; fig. 1 to 7<br>(Family: none) | 1-22 |
| P,X | JP 2013-230319 A  (Olympus Corp.),<br>14 November 2013 (14.11.2013),<br>entire text; fig. 1 to 15<br>(Family: none) | 1-22 |

Form PCT/ISA/210 (continuation of second sheet) (July 2009)

**INTERNATIONAL SEARCH REPORT**

International application No.

PCT/JP2013/075625

| Box No. II    Observations where certain claims were found unsearchable (Continuation of item 2 of first sheet) |
|---|

This international search report has not been established in respect of certain claims under Article 17(2)(a) for the following reasons:

1. ☒ Claims Nos.: 23
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III    Observations where unity of invention is lacking (Continuation of item 3 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/075625

Continuation of Box No.II-1 of continuation of first sheet(2)

Claim 23 pertains to a method for controlling an endoscope device, the method including a step for controlling a focusing operation of an imaging optical system, the step being a step in which a device is used inside the human body. Consequently, the method falls under the category of methods for treatment of the human body by surgery or therapy and relates to a subject matter for which the International Searching Authority is not required, under the provisions of PCT Article 17(2)(a)(i) and [PCT Rule 39.1(iv)], to make an international search.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8106060 A